# FASCICULE DE BREVET EUROPEEN

(11) **EP 3 197 460 B1**
(45) Date de publication et mention de la délivrance du brevet: **20.04.2022**
(21) Numéro de dépôt: 15788100.4
(22) Date de dépôt: 22.09.2015
(51) Int. Cl.: A61K 31/616, A61P 17/00, A61K 9/06, A61K 45/06, A61K 8/37

(54) **UTILISATION DERMOCOSMETIQUE OU PHARMACEUTIQUE D'UNE COMPOSITION CONTENANT AU MOINS UN INHIBITEUR DE CERTAINES CHIMIOKINES**
DERMOKOSMETISCHE ODER PHARMAZEUTISCHE VERWENDUNG EINER ZUSAMMENSETZUNG MIT MINDESTENS EINEM INHIBITOR BESTIMMTER CHEMOKINE
DERMOCOSMETIC OR PHARMACEUTICAL USE OF A COMPOSITION CONTAINING AT LEAST ONE INHIBITOR OF CERTAIN CHEMOKINES

(30) Priorité: 24.09.2014 FR 1402163
(43) Date de publication de la demande: 02.08.2017
(73) Titulaire: Greenpharma, 45100 Orléans (FR); Centre National de la Recherche Scientifique (CNRS), 75794 Paris Cedex 16 (FR); Université de Strasbourg, 67081 Strasbourg (FR)
(72) Inventeur: BERNARD, Philippe, F-45240 La Ferté Saint-Aubin (FR); ABBOUD, Dayana, F-67400 Illkirch (FR); GALZI, Jean-Luc, F-67500 Weitbruch (FR); FROSSARD, Nelly, F-67000 Strasbourg (FR); PILLARD, Christelle, F-45100 Orléans (FR); DO, Quoc Tuan, F-45100 Orléans (FR)
(74) Mandataire: Vial, Lionel
(86) Numéro de dépôt international: PCT/FR2015/000185
(87) Numéro de publication internationale: WO 2016/046456

(56) Documents cités:
- WO-A1-2013/083825
- US-A1- 2014 274 974
- BALTHAZAR-LETAWE D: "Clinical evaluation of a salicylate emulsion for topical use in physical medicine", ARS MEDICI REVUE INTERNATIONALE DE THERAPIE PRATIQUE 1979 BE, vol. 34, no. 20, 1979, pages 1799-1803, XP009184119, ISSN: 0374-5783
- A. F. ARTAMONOV ET AL: "Synthesis of [alpha]-monoglycerides of aromatic acids", CHEMISTRY OF NATURAL COMPOUNDS, vol. 35, no. 4, 1 juillet 1999 (1999-07-01), pages 404-408, XP055090710, ISSN: 0009-3130, DOI: 10.1007/BF02282504

## Description

La présente invention concerne l'utilisation de certains dérivés inhibant les cytokines chimio-attractantes (généralement appelées chimiokines), dans des compositions cosmétiques ou pharmaceutiques. Plus précisément, elle se rapporte à de nouvelles applications desdits dérivés de l'acide salicylique ou nicotinique pour traiter des maladies inflammatoires chroniques ; on peut citer à cet égard, les maladies inflammatoires chroniques externes comme les dermatoses (acné, dermatite atopique, psoriasis, eczéma, peau sèche à tendance atopique, rougeurs...) ou internes comme la maladie de Crohn, l'asthme, l'asthme allergique et la rhinite allergique.

La maladie de Crohn est une maladie inflammatoire chronique de l'intestin. La publication de Grip O. et Janciauskiene S. (Atorvastatin reduces plasma levels of chemokine (CXCL10) in patients with Crohn's disease, PloS One, 2009, 4(5):e5263) montre l'importance des chimiokines dans cette pathologie.

La rhinite allergique est une maladie chronique souvent attachée aux dermatoses chroniques. Un article de Takeuchi S. et al (Changes in thymus- and activation-regulated chemokine (TARC) associated with allergen immunotherapy in patients with perennial allergie rhinitis. J Investig Allergol Clin Immunol, 2005, 15(3), 172-6) montre l'implication des chimiokines dans cette pathologie.

Les dermatoses sont des affections de la peau et des muqueuses, qui se caractérisent par des manifestations inesthétiques comme des rougeurs et des plaques de desquamation. Plusieurs pathologies sont regroupées sous la dénomination de dermatoses. On peut citer, à titre d'exemples non limitatifs, l'eczéma, la dermatite atopique, le psoriasis, les dermites séborrhéiques ou encore l'acné. Ces dermatoses résultent bien souvent de phénomènes inflammatoires et de désordres immunitaires.

Dans le cas de la dermatite atopique, les lésions se caractérisent par une sécheresse cutanée importante et par des lésions inflammatoires : éruptions érythémateuses papuleuses, vésiculeuses, squameuses et très prurigineuses (démangeaisons). Sur le plan histologique, la dermatite atopique se caractérise, comme bien des dermatoses, par une infiltration de lymphocytes, monocytes et de polynucléaires éosinophiles autour des petits vaisseaux et des capillaires. Sur le plan biochimique, il a été montré que les chimiokines sont fortement impliquées dans les dermatoses et dans les maladies inflammatoires chroniques en général. A titre d'exemple, la publication de Folsgaard et al (Cord blood Th2-related chemokine CCL22 levels associate with elevated total-IgE during preschool age, Clin. Exp. Allergy, 2012, 42(11), 1596-603) met en évidence l'importance de la chimiokine CCL22 dans la dermatite atopique. Une autre publication de Hashimoto et al (Macrophage-derived chemokine (MDC)/CCL22 produced by monocyte derived dendritic cells reflects the disease activity in patients with atopic dermatitis, Journal of Dermatological Science, 2006, 44(2), 93-9) met en évidence l'importance de la même chimiokine CCL22 dans les maladies de la peau.

Les chimiokines sont une famille de petites protéines, majoritairement solubles, de 8-14 kD. Leur fonction la plus étudiée est l'attraction et le contrôle de l'état d'activation des cellules du système immunitaire. Certaines chimiokines s'avèrent être de bonnes cibles thérapeutiques pour traiter les maladies inflammatoires chroniques internes et externes chez l'homme ou l'animal.

La présente invention met en évidence de nouvelles applications cosmétiques, pharmaceutiques et vétérinaires de certains agents efficaces pour inhiber directement les chimiokines et traiter les maladies inflammatoires chroniques chez l'homme ou l'animal. Dans cette demande de brevet, on utilise le terme générique « pharmaceutique » pour définir aussi bien un produit destiné à une application chez l'homme qu'un produit destiné à une application chez l'animal.

L'invention concerne l'utilisation d'une composition dermo-cosmétique ou pharmaceutique destinée à traiter des maladies inflammatoires de la peau humaine ou animale, caractérisée en ce que ladite composition contient, dans un véhicule pharmaceutiquement acceptable, une quantité efficace d'au moins un agent actif répondant à la formule générale (I) : 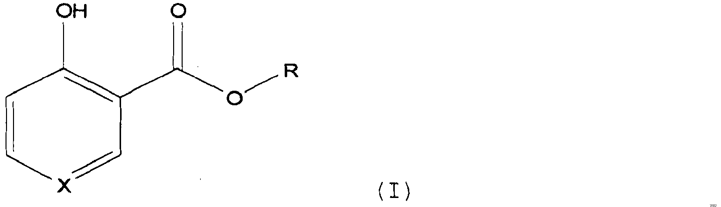 ledit composé étant choisi dans le groupe formé par 2,3-dihydroxypropyl 2-hydroxybenzoate, (S)-2,3-dihydroxypropyl 2-hydroxybenzoate, et (R)-2,3-dihydroxypropyl 2-hydroxybenzoate, ledit composé de formule (I) pouvant être sous forme d'un sel d'addition basique ou acide.

Lorsque dans la composition mise en œuvre dans l'utilisation selon l'invention, l'agent actif est sous la forme d'un sel d'addition acide, ledit acide est avantageusement choisi dans le groupe formé par les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, méthane- ou éthane-sulfonique et camphorique.

Lorsque, dans la composition mise en œuvre dans l'utilisation selon l'invention, l'agent actif est sous la forme d'un sel d'addition basique, ladite base est avantageusement choisie dans le groupe formé par l'hydroxyde de sodium ou de potassium, la triéthylamine et la tertiobutylamine.

Les composés de formule (I) peuvent posséder un ou plusieurs centre(s) asymétrique(s) et peuvent alors être isolés sous une forme optiquement active ou sous forme de leur mélange racémique. Les méthodes permettant d'obtenir des formes optiquement actives, par exemple par résolution d'une forme racémique ou par synthèse utilisant des produits de départ racémiques, sont bien connues de l'homme de l'art.

Pour la définition des composés de formule (I), le terme « alkyle » désigne un radical hydrocarboné linéaire ou ramifié ayant avantageusement de 1 à 6 atomes de carbone, tels que méthyle, éthyle, propyle, isopropyle, butyle, isobutyle, tert-butyle, pentyle, néopentyle, n-hexyle. Les groupes alkyle peuvent être substitués par un ou plusieurs groupements hydroxyle et/ou par un ou plusieurs groupements alcoxy tel que défini ci-après.

Le terme « cycloalkyle » désigne un système hydrocarboné cyclique, comprenant de 3 à 6 atomes de carbone et pouvant être mono- ou poly-cyclique. On peut citer notamment les radicaux cyclopropyle et cyclohexyle.

Les groupes « alcoxy » correspondent aux groupes alkyle linéaires ou ramifiés définis ci-dessus reliés par l'intermédiaire d'une liaison -O- (éther). On préfère tout particulièrement les groupes méthoxy, éthoxy, *n*-propyloxy, *i*-propyloxy, *n*-butoxy, *s*-butoxy, *t*-butoxy, *n*-pentoxy et *s-*pentoxy. Les groupes alcoxy peuvent être substitués par un groupe alkyle comme défini ci-dessus ou par un autre group alcoxy.

Parmi les composés de formule (I) utilisables comme agent actif dans les compositions mises en œuvre dans l'utilisation selon l'invention, on peut notamment citer : 2,3-dihydroxypropyl 2-hydroxybenzoate (codé GPN000136) (*S*)-2,3-dihydroxypropyl 2-hydroxybenzoate (référencé GPS008506) (*R*)-2,3-dihydroxypropyl 2-hydroxybenzoate (référencé GPS008505)

L'invention concerne notamment une utilisation qui met en œuvre une composition comportant au moins un composé de formule (I) comme agent actif pour traiter des maladies inflammatoires de la peau. Une telle composition peut aussi comporter au moins un agent actif autre que ceux de formule (I) pour exercer au moins une action complémentaire ou synergique.

Selon un autre aspect de l'invention, la composition mise en œuvre selon l'invention se présente sous forme de gélules, crème, gel, lotion, lait, émulsion, émulsion bi-phasique, émulsion H/E ou E/H, émulsion triphasique, solution, onguent, huile corporelle, shampoing, savon, bâton protecteur des lèvres, bâton et crayon pour maquillage, masque, nano-capsule, liposome, patchs transdermiques pour applications topiques.

Sous la forme de gel, la composition mise en œuvre selon l'invention peut comprendre des excipients appropriés, tels que les esters de cellulose, ou d'autres agents gélifiants, tels que le polymère carboxylique « Carbopol^{®} » et la gomme de guar, par exemple.

Sous la forme d'émulsions, les compositions mises en œuvre selon l'invention jouissent d'une bonne stabilité et peuvent être conservées pendant le temps nécessaire pour l'utilisation à des températures comprises entre 0 et 50 °C, sans qu'il y ait sédimentation des constituants ou séparation des phases.

Selon un autre aspect de l'invention, dans la composition mise en œuvre selon l'invention, l'(ou les) agent (s) actif (s) est (sont) mis en place dans des moyens d'encapsulation choisis dans le groupe formé par des microsphères, des liposomes, des glycosphères, des chylomicrons, des macro- micro-, et nanoparticules, des macro-, micro- et nanocapsules.

Selon un autre aspect de l'invention, dans la composition mise en œuvre selon l'invention, l'(ou les) agent(s) actif(s) est (sont) absorbé(s) ou adsorbé(s) sur des polymères organiques poudreux, des talcs, de la bentonite ou d'autres supports minéraux en poudre.

Selon un autre aspect de l'invention, dans la composition mise en œuvre selon l'invention, 1'(ou les) agent(s) actif(s) de formule (I) constitue(nt) de 0,01% à 5% en poids par rapport au poids total de la composition.

Selon un autre aspect de l'invention, dans la composition mise en œuvre selon l'invention, l'(ou les) agent (s) actif (s) de formule (I) est (ou sont) sous forme encapsulée et constitue (nt), de préférence de 0,5 à 5% en poids par rapport au poids total de la composition.

L'invention a ,notamment, pour objet l'utilisation d'une composition telle que précédemment décrite pour prévenir et/ou traiter des maladies inflammatoires chroniques soit internes, comme la maladie de Crohn, l'asthme, l'asthme allergique et la rhinite allergique, soit externes liées à des activités séborrhéiques, acnéiques, inflammatoires et immunologiques ; une telle composition peut notamment être utilisée pour prévenir et/ou traiter des dermatoses, le psoriasis, la dermatite atopique, ainsi que des états inflammatoires des systèmes cutanés ou pileux induisant des démangeaisons, des irritations, des rougeurs chroniques ou des troubles fonctionnels de la fragilité capillaire.

L'invention a donc mis en évidence la possibilité d'utiliser les composés de formule (I) comme agent(s) actif(s). Ces composés présentent de très bonnes propriétés pour inhiber certaines chimiokines, comme CCL22 et/ou CCL17.

La présente invention vise donc précisément à proposer une nouvelle utilisation pharmaceutique, dermatologique ou cosmétique mettant en œuvre des compositions capables de traiter les maladies inflammatoires chroniques internes et externes comme les peaux irritées, lésées ou sensibilisées par des agressions externes ou internes, en général, et des dermatoses, en particulier.

Ainsi, la présente invention a pour objet une utilisation cosmétique d'au moins un composé de formule (I) pour inhiber la chimiokine CCL22 et/ou CCL17, pour apaiser les peaux sèches à tendance atopique et/ou pour améliorer et/ou renforcer l'hydratation de la peau. Elle concerne donc des applications pour soigner les pathologies chroniques inflammatoires comme la maladie de Crohn, la rhinite allergique, l'asthme, les dermatoses comme la dermatite atopique, l'eczéma et le psoriasis.

Dans la visée thérapeutique de l'invention, les dérivés de formule (I) capables d'inhiber la chimiokine CCL22 et/ou CCL17 peuvent être administrés par voie topique mais ils peuvent aussi être administrés par voie orale. Ces dérivés peuvent être utilisés tels quels, sous forme liquide ou en poudre, non purifiés ou purifiés.

Les dérivés de formule (I) capables d'inhiber les chimiokines peuvent, dans les compositions mises en œuvre, être associés entres eux ou avec d'autres composés venant compléter l'effet voire synergiser cet effet. L'activité protectrice des dérivés, de formule (I) vis-à-vis des radicaux libres et des UV est intéressante dans le domaine capillaire, notamment dans le cas d'association avec des substances facilitant le bon état du cuir chevelu et du cheveu. On peut citer les associations avec des mucopolysaccharides, des minéraux, des vitamines, des céramides, des huiles végétales, des substances antiradicalaires, des filtres U.V., des acides de fleurs ou de fruits.

De même, l'activité réparatrice des composés de formule (I) capable d'inhiber les chimiokines est particulièrement intéressante, quand ils sont associés avec des substances ayant un effet cicatrisant comme des protéines, l'acide hyaluronique, des acides aminés, ou avec des substances anti-inflammatoires, anti-âge, après-solaire anti-acné ou anti-dermatose.

Ainsi, les compositions mises en œuvre selon l'invention sont tout particulièrement adaptées à une application topique pour prévenir et/ou traiter de nombreuses altérations cutanées, notamment comme agent réparateur et/ou protecteur de la peau et du système pileux comme les cheveux, pour lutter contre les agressions externes liées à la pollution, au soleil, au stress oxydatif, au vieillissement et aux pathologies cutanées entrainant un dysfonctionnement de l'homéostasie de l'épiderme ou des cheveux.

Ces compositions cosmétiques mises en œuvre selon l'invention peuvent aussi prendre la forme de lotions ou solutions dans lesquelles les composés de formule (I) sont sous forme encapsulée, par exemple dans des microsphères. Ces microsphères peuvent, par exemple, être constituées de corps gras, d'agar et d'eau. Les agents actifs peuvent être aussi incorporés dans des vecteurs de type liposomes, glycosphères, dans des chylomicrons, des macro-, micro-, nanoparticules ainsi que les macro-, micro- et nanocapsules et aussi être adsorbés sur des polymères organiques poudreux, les talcs, bentonites et autres supports minéraux.

Pour la préparation des compositions mises en œuvre selon l'invention, les composés de formule (I) peuvent être mélangés aux excipients généralement employés en cosmétique. Les compositions cosmétiques mises en œuvre selon l'invention peuvent donc contenir des additifs ou des adjuvants usuels en cosmétologie, comme par exemple des agents antibactériens ou des parfums mais aussi des lipides d'extraction et/ou de synthèse, des polymères gélifiants et viscosants, des tensio-actifs, des émulsifiants, des actifs hydro- ou lipo-solubles, des extraits de plantes, des extraits tissulaires, des extraits marins, ou des actifs de synthèse.

L'utilisation dermo-cosmétique ou pharmaceutique des composés de formule (I) comprend tous les produits de soin du corps et de la peau, y compris les produits solaires, protecteurs et bronzants, les produits anti-âge, anti-séborrhéiques, toniques, les produits assurant l'amélioration de l'aspect de la peau y compris le traitement acnéique, le traitement des rougeurs cutanées, le traitement du cuir chevelu et celui de la chute des cheveux.

Les compositions cosmétiques mises en œuvre selon la présente invention peuvent aussi comprendre d'autres agents actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, ainsi que d'autres agents actifs ayant une action sur la tonicité cutanée et la protection du cheveu.

Les compositions cosmétiques mises en œuvre selon la présente invention sont, de préférence, à utiliser quotidiennement en les appliquant une ou plusieurs fois par jour.

Les compositions cosmétiques mises en œuvre selon la présente invention sont très bien tolérées, elles ne présentent aucune phototoxicité et leur application sur la peau, pour des périodes de temps prolongées, n'implique aucun effet systémique.

L'invention concerne aussi l'utilisation de composés de formule (I) pour la préparation de compositions pharmaceutiques présentant une activité anti-inflammatoire et/ou dermoprotectrice. Ces compositions sont utiles pour prévenir et/ou traiter notamment des maladies dermatologiques liées à des activités séborrhéiques, acnéiques, inflammatoires et immunologiques.

En résumé, l'invention se rapporte donc à l'utilisation d'une composition pharmaceutique, dermatologique ou cosmétique, contenant des composés de formule (I) capables d'inhiber certaines chimiokines, destinées à prévenir et/ou traiter les pathologies et désordres résultant :
du vieillissement naturel ou prématuré de la peau ou des cheveux ;
de dermatoses, comme le psoriasis, l'acné et la dermatite atopique ;
d'états inflammatoires et/ou immunitaires des systèmes cutanés ou pileux, induisant des démangeaisons, irritations, rougeurs chroniques, irritations et démangeaisons persistantes, et des troubles fonctionnels de la fragilité capillaire.

La présente invention est maintenant illustrée par les exemples donnés ci-après.

Les exemples 1 à 3 illustrent un procédé de préparation de composés de formule (I) capable d'inhiber la chimiokine CCL22 selon l'invention.

Les produits de départ sont disponibles dans le commerce ou peuvent être synthétisés par des méthodes classiques connues de l'homme du métier.

### Exemple 1 :

### Synthèse du 2,3-dihydroxypropyl 2-hydroxybenzoate (codé GPN136)

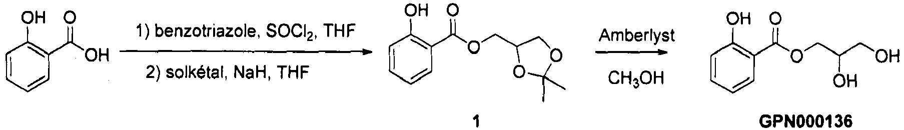

Dans un ballon sous argon, 2.17 g (3.15 éq.) de 1H-benzotriazole sont dissous dans 30 mL de tétrahydrofurane anhydre, puis 0.42 mL (1 éq.) de chlorure de thionyle sont ajoutés. Le mélange est agité pendant 45 minutes à température ambiante puis 800 mg (5.792 mmol; 1 éq.) d'acide salicylique dissous dans 20 mL de tétrahydrofurane anhydre sont additionnés. Le milieu réactionnel est agité 3 heures à température ambiante. Il est ensuite filtré rapidement sur fritté. Le filtrat est évaporé pour conduire à l'acide activé (benzotriazol-1-yl(2-hydroxyphényl) méthanone) qui est directement engagé dans l'étape suivante.

Dans un ballon sous argon, 696 mg (5.266 mmol) de solkétal sont dissous dans 10 mL de tétrahydrofurane anhydre et 232 mg (1.1 éq.) d'hydrure de sodium (60% dans l'huile) sont ajoutés. Le mélange est agité 30 minutes à température ambiante. Puis 1.1 éq. (5.792 mmol) de benzotriazol-1-yl(2-hydroxyphényl)méthanone en solution dans 20 mL de tétrahydrofurane anhydre sont ajoutés. Le milieu réactionnel est agité 5 heures à température ambiante, puis hydrolysé avec 30 mL d'eau. Il est extrait au dichlorométhane (3 fois 30 mL). Les phases organiques rassemblées sont séchées sur sulfate de magnésium, filtrées et évaporées sous pression réduite. Le brut est purifié par chromatographie sur gel de silice (éther de pétrole / acétate d'éthyle 9/1) pour conduire à 1.16 g (rendement : 87%) de produit attendu codé 1 sous forme d'une huile incolore.

RMN ¹H (250 MHz, CDCl₃) : δ 1,39 (s, 3H, CH₃) ; 1.46 (s, 3H, CH₃) ; 3.89 (dd, 1H, *J* = 5.3 et 8.7 Hz, CH₂) ; 4.16 (dd, 1H, *J* = 6.2 et 8.7 Hz, CH₂) ; 4.33 - 4.51 (m, 3H, CH₂ et CH) ; 6.89 (td, 1H, J = 1.2 et 8.0 Hz, CH Ar) ; 6.99 (dd, 1H, J = 0.9 et 8.3 Hz, CH Ar) ; 7.47 (td, 1H, J = 1.6 et 8.3 Hz, CH Ar) ; 7.87 (dd, 1H, *J* ₌ 1.6 et 8.0 Hz, CH Ar) ; 10.62 (s, 1H, OH).

Dans un ballon, 1 g (3.9640 mmol) du composé codé 1 sont dissous dans 25 mL de méthanol et 1.2 g de résine Amberlyst 15 activée sont ajoutés. Le milieu est agité pendant 19 heures à température ambiante, filtré sur fritté. La résine est rincée avec du méthanol. Le filtrat est ensuite évaporé sous pression réduite. Le brut est purifié par chromatographie sur gel de silice (éther de pétrole / acétate d'éthyle : 5/5 puis 3/7) pour conduire à 620 mg du composé codé GPN000136 (rendement : 74%) sous forme d'un solide blanc.

RMN ¹H (250 MHz, CDCl₃) : δ 2.61 (sl, 1H, OH) ; 3.07 (sl, 1H, OH) ; 3.68 - 3.78 (m, 2H, CH₂) ; 4.05 - 4.15 (m, 1H, CH) ; 4.39 - 4.51 (m, 2H, CH₂) ; 6.88 (td, 1H, J = 1.0 et 7.6 Hz, CH Ar) ; 6.98 (dd, 1H, *J* = 0.9 et 8.4 Hz, CH Ar) ; 7.46 (td, 1H, J = 1.7 et 7.7 Hz, CH Ar) ; 7.84 (dd, 1H, *J* = 1.7 et 8.0 Hz, CH Ar) ; 10.57 (s, 1H, OH).

RMN ¹³C (62.5 MHz, CDCl₃) : 63.5 (CH₂) ; 65.6 (CH₂) ; 70.2 (CH) ; 112.1 (Cq) ; 117.9 (CH) ; 119.4 (CH) ; 130.0 (CH) ; 136.2 (CH) ; 161.8 (Cq) ; 170.3 (Cq).

### Exemple 2 : Synthèse du (S)-2,3-dihydroxypropyl 2-hydroxybenzoate (codé GPS008506)

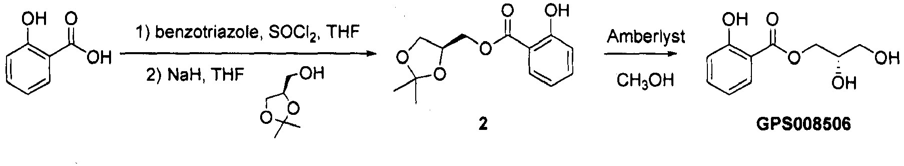

Dans un ballon sous argon, 0.3 mL (2.4194 mmol) de (R)- 2,3-*O*-isopropylidéne glycérol sont dissous dans 5 mL de tétrahydrofurane anhydre et 106 mg (1.1 éq.) d'hydrure de sodium (60% dans l'huile) sont ajoutés. Le mélange est agité pendant 30 minutes à température ambiante. Puis 637 mg (1.1 éq.) d'acide activé décrit précédemment (benzotriazol-1-yl(2-hydroxyphenyl) methanone) dissous dans 12 mL de tétrahydrofurane anhydre sont additionnés. Le milieu réactionnel est agité 4 heures à température ambiante puis hydrolysé avec 40 mL d'eau. Il est extrait au dichlorométhane (3 fois 30 mL). Les phases organiques rassemblées sont séchées sur sulfate de magnésium, filtrées et évaporées sous pression réduite. Le brut est purifié par chromatographie sur gel de silice (éther de pétrole / acétate d'éthyle 9/1) pour conduire à 520 mg (rendement : 85%) du produit attendu (codé 2) sous forme d'une huile incolore.

RMN ¹H (250 MHz, CDCl₃) : δ 1,39 (s, 3H, CH₃) ; 1.46 (s, 3H, CH₃) ; 3.89 (dd, 1H, *J* = 5.3 et 8.7 Hz, CH₂) ; 4.16 (dd, 1H, J = 6.2 et 8.7 Hz, CH₂) ; 4.33 - 4.51 (m, 3H, CH₂ et CH) ; 6.89 (td, 1H, J = 1.2 et 8.0 Hz, CH Ar) ; 6.99 (dd, 1H, J = 0.9 et 8.3 Hz, CH Ar) ; 7.47 (td, 1H, J = 1.6 et 8.3 Hz, CH Ar) ; 7.87 (dd, 1H, J = 1.6 et 8.0 Hz, CH Ar) ; 10.62 (s, 1H, OH).

Dans un ballon, 650 mg (2.5766 mmol) du composé codé 2 sont dissouts dans 10 mL de méthanol et 800 mg de résine Amberlyst 15 activée sont ajoutés. Le milieu est agité 24 heures à température ambiante, filtré sur fritté. La résine est rincée avec du méthanol. Le filtrat est ensuite évaporé sous pression réduite. Le brut est purifié par chromatographie sur gel de silice (éther de pétrole / acétate d'éthyle : 6/4, 4/6 puis 3/7) pour conduire à 440 mg du composé référencé GPS008506 (rendement : 80%) sous forme d'un solide blanc. Pouvoir rotatoire : α_{D} = +15.5 (c = 1 ; EtOH).

RMN ¹H (250 MHz, CDCl₃) : δ 2.61 (sl, 1H, OH) ; 3.07 (sl, 1H, OH) ; 3.68 - 3.78 (m, 2H, CH₂) ; 4.05 - 4.15 (m, 1H, CH) ; 4.39 - 4.51 (m, 2H, CH₂) ; 6.88 (td, 1H, J = 1.0 et 7.6 Hz, CH Ar) ; 6.98 (dd, 1H, J = 0.9 et 8.4 Hz, CH Ar) ; 7.46 (td, 1H, *J* = 1.7 et 7.7 Hz, CH Ar) ; 7.84 (dd, 1H, J = 1.7 et 8.0 Hz, CH Ar) ; 10.57 (s, 1H, OH).

RMN ¹³C (62.5 MHz, CDCl₃) : δ 63.5 (CH₂) ; 65.6 (CH₂) ; 70.2 (CH) ; 112.1 (Cq) ; 117.9 (CH) ; 119.4 (CH) ; 130.0 (CH) ; 136.2 (CH) ; 161.8 (Cq) ; 170.3 (Cq).

### Exemple 3 :

### Synthèse du (R)-2,3-dihydroxypropyl 2-hydroxybenzoate (codé GPS008505)

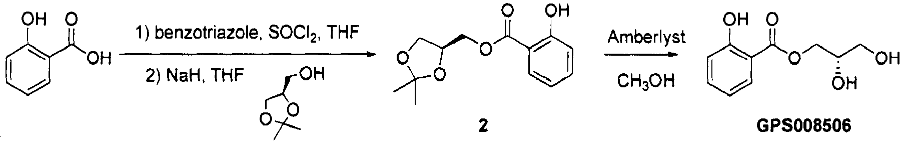

Dans un ballon sous argon, 0.4 mL (3.2385 mmol) de (S)- 2,3-O-isopropylidene glycérol sont dissous dans 7 mL de tétrahydrofurane anhydre et 142 mg (1.1 éq.) d'hydrure de sodium (60% dans l'huile) sont ajoutés. Le mélange est agité 30 minutes à température ambiante. Puis 852 mg (1.1 éq.) d'acide activé décrit précédemment (benzotriazol-1-yl(2-hydroxyphenyl) methanone) dissous dans 16 mL de tétrahydrofurane anhydre sont additionnés. Le milieu réactionnel est agité 5 heures à température ambiante puis hydrolysé avec 40 mL d'eau. Il est extrait au dichlorométhane (3 fois 30 mL). Les phases organiques rassemblées sont séchées sur sulfate de magnésium, filtrées et évaporées sous pression réduite. Le brut est purifié par chromatographie sur gel de silice (éther de pétrole / acétate d'éthyle 9/1) pour conduire à 730 mg (rendement : 89%) du produit attendu (codé 3) sous forme d'une huile incolore.

RMN ¹H (250 MHz, CDCl₃) : δ 1,39 (s, 3H, CH₃) ; 1.46 (s, 3H, CH₃) ; 3.89 (dd, 1H, *J* = 5.3 et 8.7 Hz, CH₂) ; 4.16 (dd, 1H, *J* = 6.2 et 8.7 Hz, CH₂) ; 4.33 - 4.51 (m, 3H, CH₂ et CH) ; 6.89 (td, 1H, J = 1.2 et 8.0 Hz, CH Ar) ; 6.99 (dd, 1H, J = 0.9 et 8.3 Hz, CH Ar) ; 7.47 (td, 1H, J = 1.6 et 8.3 Hz, CH Ar) ; 7.87 (dd, 1H, J = 1.6 et 8.0 Hz, CH Ar) ; 10.62 (s, 1H, OH).

Dans un ballon, 650 mg (2.5766 mmol) du composé codé 3 sont dissous dans 15 mL de méthanol et 800 mg de résine Amberlyst 15 activée sont ajoutés. Le milieu est agité 24 heures à température ambiante, filtré sur fritté. La résine est rincée avec du méthanol. Le filtrat est ensuite évaporé sous pression réduite. Le brut est purifié par chromatographie sur gel de silice (éther de pétrole / acétate d'éthyle : 5/5 puis 3/7) pour conduire à 480 mg du composé référencé GPS008505 (rendement : 87%) sous forme d'un solide blanc. Pouvoir rotatoire : α_{D} = -16.6 (c = 1 ; EtOH).

RMN ¹H (250 MHz, CDCl₃) : δ 2.61 (si, 1H, OH) ; 3.07 (sl, 1H, OH) ; 3.68 - 3.78 (m, 2H, CH₂) ; 4.05 - 4.15 (m, 1H, CH) ; 4.39 - 4.51 (m, 2H, CH₂) ; 6.88 (td, 1H, J = 1.0 et 7.6 Hz, CH Ar) ; 6.98 (dd, 1H, *J* = 0.9 et 8.4 Hz, CH Ar) ; 7.46 (td, 1H, J = 1.7 et 7.7 Hz, CH Ar) ; 7.84 (dd, 1H, J = 1.7 et 8.0 Hz, CH Ar) ; 10.57 (s, 1H, OH).

RMN ¹³C (62.5 MHz, CDCl₃) : δ 63.5 (CH₂) ; 65.6 (CH₂) ; 70.2 (CH) ; 112.1 (Cq) ; 117.9 (CH) ; 119.4 (CH) ; 130.0 (CH) ; 136.2 (CH) ; 161.8 (Cq) ; 170.3 (Cq).

L'exemple 4 met en évidence le protocole technique permettant d'enregistrer les effets des produits selon l'invention sur les chimiokines.

### Exemple 4 :

Propriétés inhibitrices des dérivés de formule (I) sur les chimiokines.

### Cellules exprimant le récepteur CCR4 des chimiokines

Afin de permettre un enregistrement aisé des réponses associées au récepteur CCR4, le cDNA qui permet son expression est cloné dans le plasmide pIRES (ClonTech) en fusion avec la protéine GFP, selon Vollmer et al. (1999), ce qui permet de mesurer aisément l'expression du récepteur chimérique EGFP-CCR4. Les cellules HEK 293 (ATCC) sont cultivées dans le milieu minimum essentiel MEM (Invitrogen) en présence de 10% de sérum de veau fœtal (Gibco-BRL), 100 U/mL penicilline (Invitrogen), 100 αg/mL streptomycine (Invitrogen) et 2 mM L-glutamine (Invitrogen) à 37°C dans atmosphère saturée en eau et contenant 5% CO2. Les cellules sont transfectées selon la méthode du précipité de phosphate de calcium de Chen et Okayama, 1986, et les cellules ayant incorporé le plasmide d'expression sont sélectionnées à l'aide de 600 αg/mL de geneticine G-418 (PAA) pendant 5 semaines. Les clones sont sélectionnés après avoir été caractérisés par microscopie de fluorescence et analyse cytometrique au FACS (fluorescence-assisted cell sorting).

### Construction de la protéine recombinante Gqi5

Afin de permettre un enregistrement aisé des réponses cellulaires associées au récepteur CCR4, on modifie son couplage naturel à l'inhibition de production d'AMPc vers un couplage à la phospholipase C qui permet des mesures de calcium. On produit dans ce but la protéine chimérique Gqi5 par mutagénèse dirigée des 5 derniers acides aminés de Gq que l'on remplace par leurs homologues de Gi. Les cellules exprimant le récepteur CCR4 sont transfectées par le plasmide pCNA3.1 dans lequel le cDNA codant Gqi5 a été incorporé. Les enregistrements de réponses calciques sont réalisés le lendemain de la transfection.

### Enregistrement des réponses calciques cellulaires et de leur inhibition par les molécules de la collection GPN :

Les cellules sont chargées en indicateur fluorescent du calcium, INDO-1, selon le protocole fourni par le fournisseur (Molecular probes), distribuées dans des puits de microplaques et placées dans un lecteur-pipetteur de fluorescence (FlexStation, Molecular devices). L'élévation de calcium intracellulaire est mesurée par changement d'intensité de fluorescence à 401 et 475 nm (excitation 355 nm). Des points de mesures sont enregistrés chaques 5s pendant 150s pour chaque puits. Les molécules sont testées en présence de 5 nM de chimiokine CCL22 (commerciale)

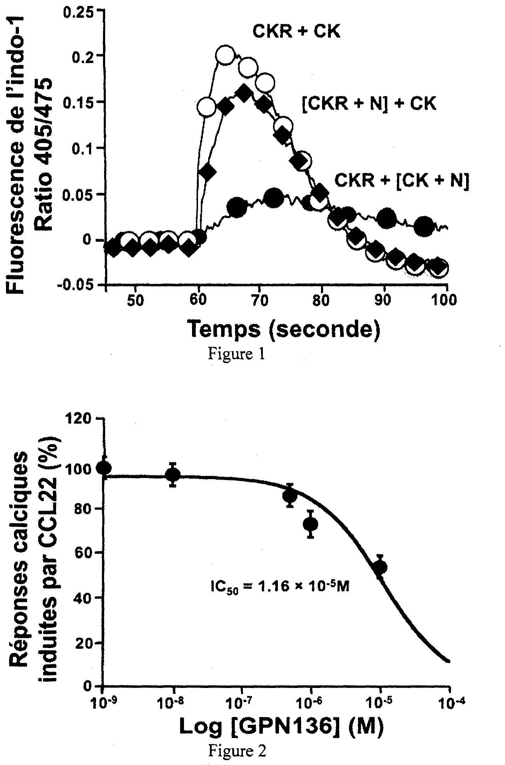

La figure 1 illustre de manière générale les résultats obtenus : l'addition de chimiokine (CK) à des cellules exprimant le récepteur de chimiokine (CKR) déclenche une libération de calcium intracellulaire recelée par le changement de fluorescence de la sonde calcique Indo-1. La réponse se développe en quelques secondes et s'atténue avec le temps pour atteindre le retour en 2 minutes environ pour une concentration de 5 à 10 nM de la chimiokine CCL22 sur des cellules HEK 293 exprimant CCR4. Si l'on préincube les molécules à tester avec la chimiokine [CK+N] et qu'on ajoute le mélange aux cellules, on observe une forte diminution de l'amplitude de la réponse si la molécule à tester est un neutraligand de la chimiokine. Inversement lorsqu'on préincube le neutraligand avec les cellules exprimant le récepteur [CKR + N] on observe une bien moindre diminution de l'amplitude de la réponse.

Ce protocole a été employé pour identifier les neutraligands de CCL22 présentés dans le tableau 1.

*L'affinité apparente des molécules de GPN136* est déterminée par l'établissement d'une relation dose-effet de l'inhibition de la réponse calcique ainsi que présenté en figure 2 et les résultats obtenus sont reportés dans le tableau 1.

### Tableau 1 : relations structure/fonction des dérivés de formule (I)

**TABLEAU 1**

| Reference | Structure | Inhibition des réponses calciques induites par CCL17 (%) | Inhibition des réponses calciques induites par CCL22 (%) |
|---|---|---|---|
| GPN136 | 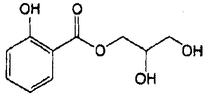 | 0 | 35±8 |
| GPS008504 | 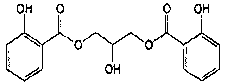 | 0 | 10±5 |
| GPS008505 | 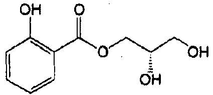 | 0 | 37±6 |
| GPS008506 | 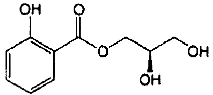 | 0 | 40±5 |
| GPS008507 | 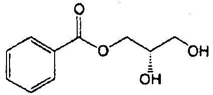 | 0 | 0 |
| GPS008508 | 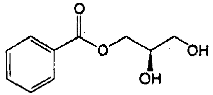 | 0 | 0 |
| Acide Acétylsalicylique | 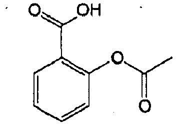 | 0 | 0 |
| GPN0001207 | 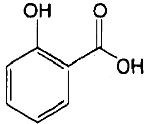 | 0 | 0 |

Les composés GPS008504, GPS008507, GPS008508, l'acide acetylsalicylique et le GPN001207 ont été utilisés comme composés de référence.

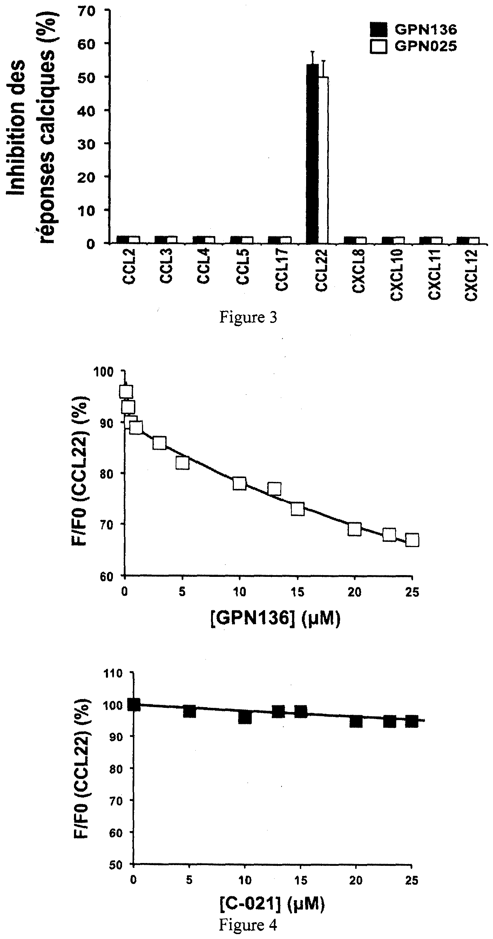

*L'analyse de la sélectivité des molécules* est réalisée par mesure d'inhibition de la réponse évoquée par d'autres chimiokines sur leurs récepteurs respectifs ainsi, dans la figure 3, les molécules ont été testée sur les paires CCL2/CCR2, CCL3-5/CCR5, CCL17/CCR4, CCL22/CCR4, CXCL8/CXCR8, CXCL10/CXCR2, CXCL11/CXCR3, CXCL12/CXCR4

La figure 3 donne la sélectivité des neutraligands de CCL22 (GPN136 (barres noires) et GPN025 (barres blanches)) sur les chimiokines indiquées et leurs récepteurs respectifs.

### Détermination de l'affinité d'interaction des neutraligands de CCL22 par extinction de fluorescence des résidus de tryptophane.

Cette mesure est réalisée comme décrit dans Hachet-Haas et al. (Small neutralizing molecules to inhibit actions of the chemokine CXCL12, J Biol Chem, 2008, 283(34), 23189-99). Brièvement : les mesures de fluorescence sont réalisées sur un spectrofluorimètre Fluorolog 3 (JobinYvon/Spex) dans une cuvette en quartz. CCL22 (1,5 µM (tampon HEPES sans albumine) dans 1 mL volume final) est excitée à 285 nm and son spectre d'émission est enregistré entre 300 et 400 nm après chaque ajout d'un aliquot de molécule à tester (1 uL). Les mesures sont effectuées à 20°C et les solutions sont agitées à l'aide d'un barreau magnétique. Les spectres d'émission enregistré »s sont corrigés par soustraction du spectre d'émission du composés à tester seul.

La figure 4 indique qu'une extinction de fluorescence de CCL22 est observée uniquement avec le neutraligand (GPN 136) et non pas avec l'antagoniste du récepteur (C-021). L'allure de la courbe de titration de CCL22 (1,5 µM) par GPN 136 est biphasique, indiquant la présence vraisemblable de plusieurs sites de liaison pour GPN 136, dont un au moins est de haute affinité. L'ajustement des traces par l'équation (RL)²+(RL)×(-Ro-Lo-KD)+Ro×Lo=0 avec (RL)=((Ro+Lo+KD)±((-Ro-Lo-KD)² -4×Ro×Lo)^{1/2})/2 permet d'estimer l'affinité pour chaque site : KD< 50 nM pour le site de haute affinité et KD= 53µM.

Il n'y a pas de signal pour la molécule C-021.

La figure 4 montre l'inhibition de la fluorescence intrinsèque de CCL22 par le neutraligand GPN 136 (carrés blancs). L'antagoniste du récepteur CCR4, C-021, ne présente pas une diminution de fluorescence du tryptophane (carrés noirs).

L'exemple 5 permet de montrer comment sont sélectionnés les candidats actifs non cytotoxiques.

### Exemple 5 :

### Propriétés des dérivés sur la migration de kératinocytes et cytotoxicité

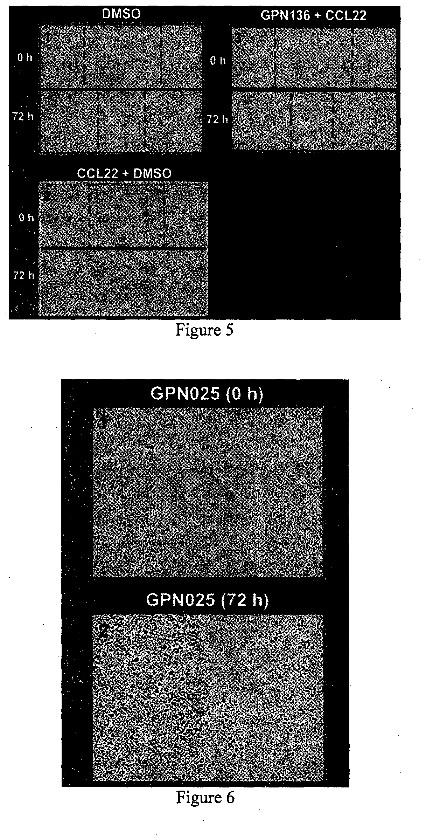

L'activité de la molécule GPN 136 a été évaluée dans un essai de migration de kératinocytes humains réalisé selon un test de cicatrisation d'un tapis cellulaire. Il est effectivement connu que, dans ces pathologies chroniques, une migration importante des cellules peut favoriser la formation de prurit, une des manifestations de la dermatose. Les kératinocytes humains immortalisés obtenus auprès du DKFZ (Heidelberg, Allemagne) sont cultivés dans du milieu de Dulbecco modifié par Eagle (DMEM, InVitrogen) complémenté par 10% de sérum de veau fœtal (Gibco-BRL) et 100 U/ml de pénicilline (Invitrogen), 100 µg/ml de streptomycine (Invitrogen)and 2 mM L glutamine(Invitrogen) à 37°C et 5% CO2. Les puits d'une plaque de culture 24 puits sont ensemencés avec 200.000 cellules et la culture est maintenue jusqu'à formation d'un tapis confluent. Le tapis est alors endommagé en effectuant un grattage linéaire à l'aide pointe de pipette stérile de 0.1-10µL. Le milieu est alors remplacé pour éliminer les cellules et leurs débris et la culture est poursuivie dans différentes conditions expérimentales : (i) sans aucun ajout, (ii) ajout de 100 ng/ml de CCL22 et (iii) ajout d'un mélange contenant 100 ng/ml de CCL22 et la molécule à tester, par exemple GPN 136. L'effet des molécules seules est aussi enregistré dans des puits différents. Afin de bloquer la prolifération cellulaire, chaque puits reçoit une dose de 1µg/ml de mytomycine (Sigma-Aldrich). Les plaques de culture sont disposées dans l'appareil IncuCyte qui enregistre la croissance du tapis cellulaire par imagerie transmise en conditions de culture cellulaire (37°C, 5%CO2, atmosphère saturée d'eau)pendant 72h. des images en contraste de phase sont acquise chaque 2 heures. La cicatrisation est définie (quantifiée) comme le rapport de largeur de la cicatrice au temps t par rapport au temps 0. L'expérience est répétée trois fois par condition. Les résultats obtenus sont rapportés dans la figure 5. La figure 5 montre la migration de keratinocytes humains sous l'effet de GNP136 ; elle montre des photographies des tapis de cellules Hacat humaines après blessure au temps 0 et après 72h de culture en présence d'excipient seul (DMSO, panneau 1), de CCL22 et d'excipient (CCL22+DMSO, panneau 2) et de CCL22 et GPN 136 (CCL22+GPN136, panneau 3)

La figure indique que la migration cellulaire est fortement accélérée par la chimiokine CCL22 qui agit sur les récepteurs CCR4 présents dans les cellules Hacat. Cet effet de la chimiokine CCL22 est aboli par la molécule GPN136. Certains composés sont cytotoxiques : exemple de la molécule GPN 025.

La figure 6 montre l'effet de la molécule GPN025 ajoutée au jour J0 après blessure du tapis de cellules Hacat. On note que les cellules semblent avoir formé des syncitia après 72h d'incubation. Il est impossible d'en distinguer les contours. La molécule GPN025 endommage les cellules Hacat. Cet effet est quantifié dans l'expérience de d'activité métabolique présentée sur la figure 6.

Le test d'activité métabolique est un test colorimétrique (WST-1 assay - Ozyme) reposant sur la mesure de l'activité de l'enzyme mitochondriale succinate-tetrazolium reductase clivant le sel de tétrazolium WST-1 ((4-[3-(4-Iodophenyl)-2-(4-nitrophenyl)-2H-5-tetrazolio]-1,3 benzene disulfonate)) en un dérivé hydrosoluble, le formazan qui est un colorant absorbant la lumière dans la fenêtre 420-480 nm. Les cellules HaCat sont distribuées à raison de 200.000 cellules par puits dans une plaque de culture à 24 puits, dans un volume final de 500 µL. Elles sont cultivées pendant 24h avant d'ajouter de la mitomycine C (1 µg/ml) et le composé à tester. La plaque de culture est incubée pendant 72h à 37°C, 5%CO2, en atmosphère saturée d'eau. Le milieu est ensuite remplacé par du milieu frais complémenté de réactif WST-1 puis incubé pendant 2 heures à 37°C. Les plaques sont agitées pour homogénéiser la couleur dans les puits et l'absorbance est mesurée à 450 nm dans un lecteur de plaques multipuits. Les mesures sont réalisées en triplicats. L'activité enzymatique requérant l'intégrité cellulaire, l'absorbance sera d'autant plus forte que l'état métabolique des cellules est bon.

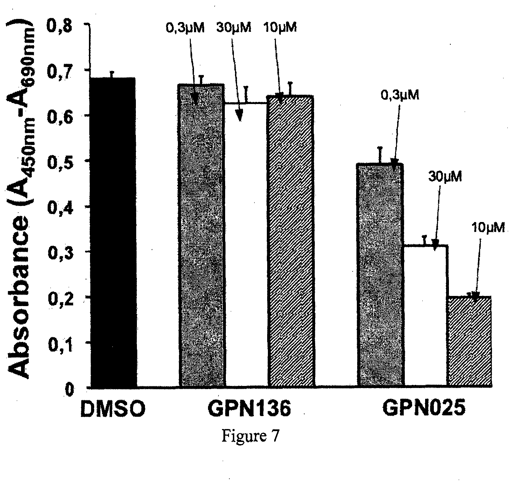

La figure 7 montre l'activité cytotoxique de GPN136 et de GPN025

Activité cytotoxique *in vitro* : les cultures de cellules HaCat sont traitées avec différentes concentrations de molécule :0,3µM (barres gris sombre) 10 µM (barres gris clair) ou 30 µM (barres blanches) des molécules GPN 136 et GPN 025. L'activité de l'enzyme formazan-réductase mitochondiale est enregistrée à l'aide du substrat chromogène WST-1 qui se transforme en produit coloré, le formazan, détecté à 450 nm. Comme le montre la figure 7, la molécule GPN 136 n'affecte pas l'activité enzymatique. En revanche, la molécule GPN 025 réduit de manière dose-dépendante et significativement le métabolisme cellulaire, révélant ainsi un effet cytotoxique de GPN 025.

### Exemple 6 :

Cet exemple présente une formulation de la composition selon l'invention.

| NOM COMMERCIAL | NOM INCI | POURCENTAGE DANS LA FORMULE |
|---|---|---|
| Simulsol 165 | PEG-100 Stearate/ Glyceryl Stearate | 3 |
| Montanov L | C14-22 Alcohol/ C12-20 Alkyl Glucoside | 2 |
| Huile de carthame | Carthamus Tinctorius Seed Oil | 2 |
| Beurre de karité | Butyrospermum Parkii Butter | 4 |
| Céramidone | Octyldodecyl PCA | 3 |
| Phytosqualane | Squalane | 5 |
| DUB MCT 5545 | Caprylic/ Capric Triglycerides | 7 |
| Cire d'abeille | Cera Alba | 1 |
| Acide sorbique | Sorbic acid | 0.1 |
| Glycérine | Glycerin | 2 |
| Sorbitol | Sorbitol | 3 |
| Aristoflex AVC | Ammonium Acryloyldimethyltaurate / VP copolymer | 1.3 |
| Gomme xanthane | Xanthan gum | 0.2 |
| Nicotinamide | Niacinamide | 1 |
| Lipacide C8G | Capryloyl glycine | 0.5 |
| Eau purifié | Aqua | Qsp pour 100 |
| GPN136 | - | 3 |

On applique cette composition tous les jours pendant 1 mois sur la peau du visage d'un homme de 20 ans souffrant de dermatite atopique sur l'ensemble du visage; cette application est faite à raison d'environ 2 g/jour pour le visage complet. On constate la disparition progressive totale des zones d'inflammation à la fin du traitement.

On a proposé ci-après des ingrédients pouvant être associés avec les compositions pour application topique afin d'amplifier l'effet ou atteindre des synergies d'action, notamment avec la formulation du présent exemple 6 (les quantités sont données en pourcentage en poids) :
a) des ingrédients ayant des propriétés anti-inflammatoires couramment utilisés dans les produits cosmétiques notamment le nicotinamide (1 à 4%), l'acide 18 béta glycyrrhétinique (0.01 à 1%), l'alpha bisabolol (0.1 à 1%) ;
b) la glycine ayant un effet apaisant (de 1 à 3%) ;
c) les micro-particules d'argent (0.1%) qui permettent de réguler, stabiliser la flore microbienne et éviter la surinfection ;
d) un agent anti-adhésion bactérienne pour limiter l'adhésion et la prolifération de Staphylococcus aureus comme le Téflose^{®} de Solabia composé de propanediol, rhamnose, glucose et acide glucuronique (2 à 4%) ;
e) les insaponifiables de beurre de karité et de tournesol pour renforcer la barrière cutanée et améliorer l'hydratation de la peau. (0.5 à 2%).
f) un extrait de chicorée de 1 à 3% (comme la Védérine^{®} de Silab) pour le renforcement et la récupération de la barrière cutanée ;
g) un dérivé d'huile de pépins de framboise et de succinate de vitamine E (Raspberry seed oil/ Tocopheryl succinate/ Aminopropanediol esters) à 1% comme le Vitaskin^{®} E de Solabia ;
h) l'octyldodécyl PCA dosé de 0.5 à 5% pour stimuler la synthèse des lipides épidermiques ;
i) une huile végétale de carthame ou onagre stabilisée sous une forme céramide-like comme l' »Omega 6 Ceramide^{®} Safflower » et l' »Omega 6 Ceramide^{®} Evening primrose » de Solabia pour améliorer la cohésion cellulaire (0.1 à1%) ;
j) un mélange optimisé d'huile d'argan, de beurre de karité et de cire d'orge, comme dans le « Stimutex^{®} AS » de DSM, pour une action anti-inflammatoire et pour diminuer la libération d'histamine (2 à 5%);
k) un mélange d'extrait d'écorce de bouleau blanc et de *Scrophularia nodosa,* comme dans le Protectol^{®} de Greentech, pour ses propriétés anti-inflammatoires du fait de la présence d'acide bétulinique (1 et 3%);
l) un mélange de lactose et de protéine de lait à 0.5% comme dans le Modukine^{™} de CLR pour ses propriétés anti-inflammatoires.

## Revendications

1. Composition dermo-cosmétique ou pharmaceutique pour une utilisation pour inhiber les chimiokines et traiter des maladies inflammatoires chroniques,
**caractérisée en ce que** ladite composition contient, dans un véhicule acceptable, une quantité efficace d'au moins un agent actif constitué par un composé répondant à la formule générale (I) : 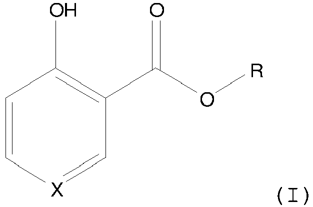 ledit composé étant choisi dans le groupe formé par 2,3-dihydroxypropyl 2-hydroxybenzoate, (*S*)-2,3-dihydroxypropyl 2-hydroxybenzoate, et (*R*)-2,3-dihydroxypropyl 2-hydroxybenzoate,ledit agent actif pouvant être sous forme de sel.

2. Composition pour une utilisation selon la revendication 1, **caractérisée en ce que**, dans la composition, l'agent actif est un sel d'addition acide, ledit acide étant choisi dans le groupe formé par les acides chlorhydrique, bromhydrique, sulfurique phosphorique, acétique, trifluoacétique, lactique, pyruvique, malonique, succinique, glutarique, fumarique, tartrique, maléique, citrique, ascorbique, méthane- ou éthane-sulfonique et camphorique.

3. Composition pour une utilisation selon la revendication 1, **caractérisée en ce que**, dans la composition, l'agent actif est un sel d'addition basique, ladite base étant choisie dans le groupe formé par l'hydroxyde de sodium ou de potassium, la triéthylamine et la tertiobutylamine.

4. Composition pour une utilisation selon l'une des revendications 1 à 3, **caractérisée en ce que** la composition mise en œuvre contient de 0,01 à 5% en poids d'agent(s) actif(s) de formule (I) par rapport au poids total de la composition.

5. Composition pour une utilisation selon l'une des revendications 1 à 4, **caractérisée en ce que** le (ou les) agents actifs de formule (I) de la composition mise en œuvre est (sont) sous forme de poudre, ou sont absorbés sur des polymères organiques poudreux, notamment talcs ou bentonites, ou sont sous forme encapsulée.

6. Composition pour une utilisation selon la revendication 5, **caractérisée en ce que** lorsqu'un agent actif de formule (I) de la composition mise en œuvre est sous forme encapsulée, son moyen d'encapsulation est choisi dans le groupe formé par des microsphères, des liposomes, des glycosphères, des chylomicrons, des macro- micro-, et nanoparticules, des macro-, micro- et nanocapsules.

7. Composition pour une utilisation selon l'une des revendications 1 à 6, **caractérisée en ce que** la composition mise en œuvre contient au moins un agent actif complémentaire autre qu'un composé de formule (I), ledit agent complémentaire étant pris dans le groupe formé par les agents de protection solaire, les agents antirides à activité antiradicalaire, antioxydante, anti-irritante, les agents favorisant la nutrition cellulaire, la respiration cellulaire, l'hydratation cellulaire, la régénération cellulaire, les traitements anti-séborrhéiques, la tonicité cutanée, la protection des cheveux, les agents cicatrisants, l'acide hyaluronique, les acides aminés, les agents anti-âge et les agents après-soleil.

8. Composition pour une utilisation selon l'une des revendications 1 à 7, **caractérisée** en ce la composition mise en œuvre est formulée sous forme de lotion, gel, émulsion, crème ou lait, émulsion bi-phasique huile dans eau ou eau dans huile, émulsion tri-phasique, huile corporelle, shampoing, savon, masque, onguent, pommade et crayon pour maquillage, bâton protecteur des lèvres, nanocapsule, liposome ou patch transdermique pour applications topiques.

9. Composition pour une utilisation selon l'une des revendications 1 à 8, **caractérisée en ce que** la composition mise en œuvre contient au moins un produit pris dans le groupe formé par des mucopolysaccharides, des vitamines, des céramides, des huiles végétales et des agents efficaces dans les dermatoses.

10. Composition pour une utilisation selon l'une des revendications 1 à 9, **caractérisée en ce que** la composition mise en œuvre contient au moins un produit pris dans le groupe formé par des agents antibactériens, des parfums, des lipides d'extraction et/ou de synthèse, des polymères gélifiants et viscosants, des tensio-actifs, des émulsifiants, des extraits de plantes, des extraits tissulaires, des extraits marins, des actifs hydro- ou lipo-solubles, des actifs de synthèse.

11. Composition pour une utilisation selon l'une des revendications 1 à 10, **caractérisée en ce que** la composition mise en œuvre est administrable par voie topique ou orale.

12. Composition pour une utilisation selon la revendication 11, pour traiter des maladies inflammatoires chroniques soit externes, soit internes, ou pour traiter des maladies dermatologiques liées à des activités séborrhéiques, acnéiques, inflammatoires et immunologiques.

13. Composition pour une utilisation selon la revendication 12, dans laquelle les maladies inflammatoires chroniques externes sont les dermatoses, l'acné, la dermatite atopique, le psoriasis, l'eczéma, la sécheresse de la peau à tendance atopique, et les rougeurs de la peau.

14. Composition pour une utilisation selon la revendication 12, dans laquelle les maladies inflammatoires chroniques internes sont la maladie de Crohn, l'asthme, l'asthme allergique et la rhinite allergique.

15. Utilisation cosmétique d'un composé de formule générale (I) tel que défini dans l'une des revendications 1 à 3, pour inhiber la chimiokine CCL22 et/ou CCL17, pour améliorer et/ou renforcer l'hydratation de la peau.

## Patentansprüche

1. Hautkosmetische oder pharmazeutische Zubereitung zum Gebrauch zur Inhibition der Chemokine und Behandlung von chronischen Entzündungskrankheiten,
**dadurch gekennzeichnet, dass** die genannte Zubereitung in einem pharmazeutisch verträglichen Vehikel eine wirksame Menge mindestens eines Wirkstoffes enthält, der eine Verbindung darstellt, die der folgenden allgemeinen Formel (I) entspricht: 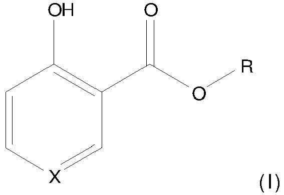 wobei die genannte Verbindung aus der Gruppe gewählt wird, bestehend aus 2,3-Dihydroxypropyl-2-hydroxybenzoat, (S)-2,3-Dihydroxypropyl-2-hydroxybenzoat, und (*R*)-2,3-Dihydroxypropyl-2-hydroxybenzoat, wobei der genannte Wirkstoff in Form eines Salzes vorliegen kann.

2. Zubereitung zum Gebrauch nach Patentanspruch 1, **dadurch gekennzeichnet, dass** der Wirkstoff der Zubereitung ein Säureadditionssalz ist, wobei die genannte Säure aus der Gruppe gewählt wird, bestehend aus Salzsäure, Bromsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Zitronensäure, Ascorbinsäure, Methan- oder Ethansulfonsäure und Kampfersäure.

3. Zubereitung zum Gebrauch nach Patentanspruch 1, **dadurch gekennzeichnet, dass** der Wirkstoff der Zubereitung ein Basenadditionssalz ist, wobei die genannte Base aus der Gruppe gewählt wird, bestehend aus Natrium- oder Kaliumhydroxid, Triethylamin und tert-Butylamin.

4. Zubereitung zum Gebrauch nach einem der Patentansprüche 1 bis 3, **dadurch gekennzeichnet, dass** die verwendete Zubereitung zwischen 0,01 und 5 Gewichtsprozent Wirkstoff(e) der Formel (I) bezogen auf das Gesamtgewicht der Zubereitung enthält.

5. Zubereitung zum Gebrauch nach einem der Patentansprüche 1 bis 4, **dadurch gekennzeichnet, dass** der/die Wirkstoff(e) der Formel (I) der verwendeten Zubereitung als Pulver vorliegt/vorliegen oder auf pulverförmige, organische Polymere, insbesondere Talke oder Bentonite, absorbiert sind, oder in verkapselter Form.

6. Zubereitung zum Gebrauch nach Patentanspruch 5, **dadurch gekennzeichnet, dass** in dem Fall, dass ein Wirkstoff der Formel (I) der verwendeten Zubereitung verkapselt vorliegt, sein Verkapselungsmittel aus der Gruppe gewählt wird, bestehend aus Mikrosphären, Liposomen, Glykosphären, Chylomikronen, Makro-, Mikro- und Nanopartikeln, Makro-, Mikro- und Nanokapseln.

7. Zubereitung zum Gebrauch nach einem der Patentansprüche 1 bis 6, **dadurch gekennzeichnet, dass** die verwendete Zubereitung mindestens einen zusätzlichen Wirkstoff, der keine Verbindung der Formel (I) ist, enthält, wobei der genannte zusätzliche Wirkstoff aus der Gruppe gewählt wird, bestehend aus Sonnenschutzmitteln, Antifaltenmitteln mit Schutz gegen freie Radikale, Antioxydationsmitteln, Antireizmitteln, Mitteln zur Förderung der Zellernährung, der Zellatmung, der Zellenhydrierung, der Zellregeneration, Mitteln für antiseborrhoische Therapie, zur Hautspannung, zum Schutz der Haare, Vernarbungsmitteln, Hyaluronsäure, Aminosäuren, Antialterungsmitteln und "After-Sun"-Mitteln.

8. Zubereitung zum Gebrauch nach einem der Patentansprüche 1 bis 7, **dadurch gekennzeichnet, dass** die verwendete Zubereitung als Lotion, Gel, Emulsion, Creme oder Milch, Öl-in-Wasser- oder Wasser-in-ÖI-Zweiphasenemulsion, Dreiphasenemulsion, Körperpflegeöl, Shampoo, Seife, Maske, Salbe, Pomade, Kosmetikstift, Lippenschutzstift, Nanokapseln, Liposomen oder transdermischer Patch zur örtlichen Anwendung formuliert ist.

9. Zubereitung zum Gebrauch nach einem der Patentansprüche 1 bis 8, **dadurch gekennzeichnet, dass** die verwendete Zubereitung mindestens ein Produkt enthält, gewählt aus der Gruppe, bestehend aus Mucopolysacchariden, Vitaminen, Ceramiden, Pflanzenölen und Wirkstoffen gegen Dermatosen.

10. Zubereitung zum Gebrauch nach einem der Patentansprüche 1 bis 9, **dadurch gekennzeichnet, dass** die verwendete Zubereitung mindestens ein Produkt enthält, gewählt aus der Gruppe, bestehend aus antibakteriellen Mitteln, Parfums, extrahierten und/oder synthetisierten Ölen, gelbildenden und Viskosität erhöhenden Polymeren, Tensiden, Emulgatoren, Pflanzenextrakten, Gewebsextrakten, Meeresextrakten, wasser- oder öllöslichen Wirkstoffen, Synthesewirkstoffen.

11. Zubereitung zum Gebrauch nach einem der Patentansprüche 1 bis 10, **dadurch gekennzeichnet, dass** die verwendete Zubereitung örtlich oder oral verbreicht werden kann.

12. Zubereitung zum Gebrauch nach Patentanspruch 11 zur Behandlung von chronischen äußeren oder inneren Entzündungskrankheiten oder zur Behandlung von Hautkrankheiten, die mit seborrhoischer, akneartiger, Entzündungs- oder Immunaktivität verbunden sind.

13. Zubereitung zum Gebrauch nach Patentanspruch 12, bei dem die chronischen äußeren Entzündungskrankheiten Dermatosen, Akne, atopische Dermatitis, Psoriasis, Ekzem, Hauttrockenheit mit atopischer Tendenz und Hautrötungen sind.

14. Zubereitung zum Gebrauch nach Patentanspruch 12, bei dem die chronischen inneren Entzündungskrankheiten Enteritis regionalis, Asthma, allergisches Asthma und allergische Rhinitis sind.

15. Kosmetischer Gebrauch einer Verbindung der allgemeinen Formel (I) nach einem der Patentansprüche 1 bis 3, zur Hemmung des Chemokins CCL22 und/oder CCL17, zur Verbesserung und/oder Erhöhung der Hautfeuchtigkeit.

## Claims

1. A dermo-cosmetic or pharmaceutical composition for use for inhibiting chemokines and treating chronic inflammatory diseases,
**characterized in that** said composition contains, in an acceptable vehicle, an effective amount of at least one active agent consisting of a compound corresponding to the general formula (I): 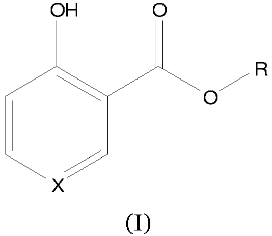 said compound being selected from the group consisting of 2,3-dihydroxypropyl 2-hydroxybenzoate, (S) -2,3-dihydroxypropyl 2-hydroxybenzoate, and (R) -2,3-dihydroxypropyl 2-hydroxybenzoate, said active agent may be in the form of salt.

2. The composition for use according to claim 1, **characterized in that**, in the composition, the active agent is an acid addition salt, said acid being chosen from the group formed by hydrochloric, hydrobromic, sulfuric, phosphoric, acetic, trifluoacetic, lactic, pyruvic, malonic, succinic, glutaric, fumaric, tartaric, maleic, citric, ascorbic, methane- or ethane-sulfonic and camphoric acids.

3. The composition for use according to claim 1, **characterized in that**, in the composition, the active agent is a basic addition salt, said base being chosen from the group formed by sodium or potassium hydroxide, triethylamine and tertiobutylamine.

4. The composition for use according to any one of claims 1 to 3, **characterized in that** the composition used contains from 0.01 to 5% by weight of active agent(s) of formula (I) relative to the total weight of the composition.

5. The composition for use according to any one of claims 1 to **4, characterized in that** the active agent(s) of formula (I) of the composition used is (are) in the form of powder, or are absorbed on powdery organic polymers, in particular talcs or bentonites, or are in encapsulated form.

6. The composition for use according to claim 5, **characterized in that** when an active agent of formula (I) of the composition used is in encapsulated form, its means of encapsulation is chosen from the group formed by microspheres, liposomes, glycospheres, chylomicrons, macro- micro-, and nanoparticles, macro-, micro- and nanocapsules.

7. The composition for use according to any one of claims 1 to 6, **characterized in that** the composition used contains at least one additional active agent other than a compound of formula (I), said additional agent being taken from the group formed by sun protection agents, anti-wrinkle agents with anti-free radical, antioxidant, anti-irritant activity, agents promoting cell nutrition, cell respiration, cell hydration, cell regeneration, anti-seborrheic treatments, skin tonicity, hair protection, healing agents, hyaluronic acid, amino acids, anti-aging agents and after-sun agents.

8. The composition for use according to any one of claims 1 to 7, **characterized in that** the composition used is formulated in the form of a lotion, gel, emulsion, cream or milk, two-phase oil-in-water or water-in-oil emulsion, tri-phase emulsion, body oil, shampoo, soap, mask, ointment, balm and pencil for makeup, protective lip stick, nanocapsule, liposome or transdermal patch for topical applications.

9. The composition for use according to any one of claims 1 to 8, **characterized in that** the composition used contains at least one product taken from the group formed by mucopolysaccharides, vitamins, ceramides, vegetable oils and effective agents in dermatoses.

10. Composition for use according to any one of claims 1 to 9, **characterized in that** the composition used contains at least one product taken from the group formed by antibacterial agents, perfumes, extraction and/or synthesis lipids, gelling and viscosity polymers, surfactants, emulsifiers, plant extracts, tissue extracts, marine extracts, water- or lipo-soluble active ingredients, synthetic active ingredients.

11. The composition for use according to any one of claims 1 to 10, **characterized in that** the composition used can be administered topically or orally.

12. The composition for use according to claim 11, for treating chronic inflammatory diseases either external or internal, or for treating dermatological diseases related to seborrheic, acne, inflammatory and immunological activities.

13. The composition for use according to claim 12, wherein the chronic external inflammatory diseases are dermatoses, acne, atopic dermatitis, psoriasis, eczema, dryness of atopic tendency skin, and redness of the skin.

14. The composition for use according to claim 12, wherein the internal chronic inflammatory diseases are Crohn's disease, asthma, allergic asthma and allergic rhinitis.

15. Cosmetic use of a compound of the general formula (I) as defined in any one of claims 1 to 3, to inhibit chemokine CCL₂₂ and/or CCL₁₇, to improve and/or enhance skin hydration.
